(19) Europäisches Patentamt
European Patent Office
Office européen des brevets

(11) EP 0 667 861 B1

(12) **EUROPÄISCHE PATENTSCHRIFT**

(45) Veröffentlichungstag und Bekanntmachung des Hinweises auf die Patenterteilung:
**19.07.2000 Patentblatt 2000/29**

(21) Anmeldenummer: **93923545.3**

(22) Anmeldetag: **25.10.1993**

(51) Int. Cl.⁷: **C07D 235/10**, C07D 491/056, C07D 405/12, A01N 43/52, A01N 43/90 // (C07D491/056, 317:00, 235:00), (C07D491/056, 319:00, 235:00), (C07D491/056, 321:00, 235:00)

(86) Internationale Anmeldenummer:
**PCT/EP93/02946**

(87) Internationale Veröffentlichungsnummer:
**WO 94/11349 (26.05.1994 Gazette 1994/12)**

(54) **2-TRIFLUORMETHYL-BENZIMIDAZOL DERIVATE, IHRE HERSTELLUNG UND IHRE VERWENDUNG ALS SCHÄDLINGSBEKÄMPFUNGSMITTEL**

2-TRIFLUOROMETHYL BENZIMIDAZOLE DERIVATIVES, THEIR PREPARATION AND THEIR USE AS PESTICIDES

DERIVES DE 2-TRIFLUOROMETHYL-BENZIMIDAZOLE, LEUR PREPARATION ET LEUR UTILISATION COMME PESTICIDES

(84) Benannte Vertragsstaaten:
**AT BE CH DE DK ES FR GB IE IT LI NL PT SE**

(30) Priorität: **06.11.1992 DE 4237557**

(43) Veröffentlichungstag der Anmeldung:
**23.08.1995 Patentblatt 1995/34**

(73) Patentinhaber: **BAYER AG**
**51368 Leverkusen (DE)**

(72) Erfinder:

- **LUNKENHEIMER, Winfried**
  **D-42115 Wuppertal (DE)**
- **BAASNER, Bernd**
  **D-51467 Bergisch Gladbach (DE)**
- **LIEB, Folker**
  **D-51375 Leverkusen (DE)**
- **BÖHM, Stefan**
  **D-51065 Köln (DE)**
- **MARHOLD, Albrecht**
  **D-51373 Leverkusen (DE)**
- **GÖRGENS, Ulrich**
  **D-40882 Ratingen (DE)**
- **STENDEL, Wilhelm**
  **D-42113 Wuppertal (DE)**
- **DEHNE, Heinz-Wilhelm**
  **D-40789 Monheim (DE)**
- **SANTEL, Hans-Joachim**
  **D-51371 Leverkusen (DE)**

(56) Entgegenhaltungen:

**EP-A- 0 251 012** **EP-A- 0 251 013**
**EP-A- 0 251 014** **EP-A- 0 260 774**
**EP-A- 0 448 206** **EP-A- 0 487 286**
**WO-A-94/11350** **DE-A- 1 545 865**
**DE-A- 1 670 786** **DE-A- 2 509 346**
**NL-A- 6 501 323**

- **CHEMICAL ABSTRACTS, vol. 66, no. 7, 13. Februar 1967, Columbus, Ohio, US; abstract no. 28771p, 'Herbicidal benzimidazoles' Seite 2743 ;Spalte 2 ;**
- **CHEMICAL ABSTRACTS, vol. 112, no. 1, 1. Januar 1990, Columbus, Ohio, US; abstract no. 7485b, '2-Polyfluoroalkyl(halo)benzimidazoles as insecticides and acaricides' Seite 736 ;Spalte 1 ;**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 667 861 B1

- CHEMICAL ABSTRACTS, vol. 116, no. 15, 13. April 1992, Columbus, Ohio, US; abstract no. 146057j, OSTROWSKI J ET AL. 'Qualitative structure-activity relationship studies on phytocidal action of benzimidazole derivatives' Seite 316 ;Spalte 1 ;

## Beschreibung

Die Erfindung betrifft neue substituierte Benzimidazole, mehrere Verfahren zu ihrer Herstellung und ihre Verwendung als Schädlingsbekämpfungsmittel.

Es ist bekannt, daß bestimmte Phosphorsäureester oder Carbamate wie beispielsweise die Verbindung O,S-Dimethyl-thiolo-phosphorsäureamid oder die Verbindung N-Methyl-O-(2-isopropoxyphenyl)-carbamat insektizide Eigenschaften besitzen (vergl. z.B. DB 12 10 835 bzw. DB 11 08 202).Aus DE-A 2 509 346 und aus NL-A 65 013 23 sind substituierte Benzimidazole und ihre Wirkung als Schädlingsbekämpfungsmittel bekannt.

Die Wirkungshöhe bzw. Wirkungsdauer dieser vorbekannten Verbindungen ist jedoch, insbesondere bei bestimmten Insekten oder bei niedrigen Anwendungskonzentrationen, nicht in allen Anwendungsgebieten völlig zufriedenstellend.

Es wurden neue substituierte Benzimidazole der folgenden allgemeinen Formel (I) gefunden:

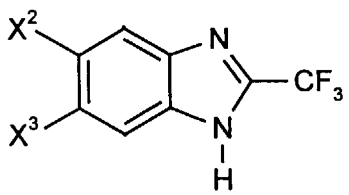

(I),

in welcher die Substituenten folgende Bedeutungen haben:

| $X^2$ | $X^3$ |
|---|---|
| $CF_3$ | Br |
| $-OCF_3$ | Cl |
| $-OCF_3$ | Br |

Es wurde gefunden, daß die neuen substituierten Benzimidazole der allgemeinen Formel (I) gute Wirksamkeit gegen Schädlinge besitzen.

Überraschenderweise zeigen die erfindungsgemäßen substituierten Benzimidazole der allgemeinen Formel (I) eine erheblich bessere insektizide Wirksamkeit im Vergleich zu den aus dem Stand der Technik bekannten aus der Gruppe der Benzimidazole welche wirkungsmäßig naheliegende Verbindungen sind.

Die 1H-Benzimidazole der Formel (I) sind erhältlich in Analogie zu bekannten Verfahren (vergl. z.B. J. Am. Chem. Soc. 75, 1292 [1953]; US-P 3.576.818).

Die Reaktionsdurchführung, Aufarbeitung und Isolierung der Produkte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Zu den oben erwähnten Schädlingen gehören:

Aus der Ordnung der Isopoda z.B. Oniscus asellus, Armadillidium vulgare, Porcellio scaber;
aus der Ordnung der Diplopoda z.B. Blaniulus guttulatus;
aus der Ordnung der Chilopoda z.B. Geophilus carpophagus, Scutigera spec.;
aus der Ordnung der Symphyla z.B. Scutigerella immaculata;
aus der Ordnung der Thysanura z.B. Lepisma saccharina;
aus der Ordnung der Collembola z.B. Onychiurus armatus;
aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria;

aus der Ordnung der Dermaptera z.B. Forficula auricularia;
aus der Ordnung der Isoptera z.B. Reticulitermes spp.;
aus der Ordnung der Anoplura z.B. Phylloxera vastatrix, Pemphigus spp., Pediculus humanus corporis, Haematopinus spp., Linognathus spp.;
aus der Ordnung der Mallophaga z.B. Trichodectes spp., Damalinea spp.;
aus der Ordnung der Thysanoptera z.B. Hercinothrips femoralis, Thrips tabaci;
aus der Ordnung der Heteroptera z.B. Eurigaster spp., Dysdercus intermedius, Piesma quadrata, Cimex lectularius, Rhodnius prolixus, Triatoma spp.;
aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.;
aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubitalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana;
aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica; aus der Ordnung der Hymenoptera z.B. Diprion spp., Hoplocampa spp., Lasius spp., Monomorium pharaonis, Vespa spp.;
aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa;
aus der Ordnung der Siphonaptera z.B. Xenopsylla cheopis. Ceratophyllus spp.; aus der Ordnung der Arachnida z.B. Scorpio maurus, Latrodectus mactans; aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus simius, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme, sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide Wirksamkeit aus.

Sie lassen sich mit besonders gutem Erfolg zur Bekämpfung von pflanzenschädigenden Insekten, wie beispielsweise gegen die Larven der Meerettichblattkäfer (Phaedon cochleariae) oder gegen die Raupen der Kohlschabe (Plutella maculipennis) oder gegen andere Plutella-Arten, wie beipielsweise Plutella xylostella oder gegen die Tabakknospenraupe (Heliothis virescens) ebenso wie zur Bekämpfung von pflanzenschädigenden Milben, wie beispielsweise gegen die gemeine Spinnmilbe (Tetranychus urticae) oder zur Bekämpfung von pflanzenschädigenden Nematoden, wie beispielsweise gegen die Nematodenart Globodera rostochiensis einsetzen.

Daneben lassen sich die erfindungsgemäßen Wirkstoffe auch zur Bekämpfung von Hygiene- und Vorratsschädlingen, wie beispielsweise gegen die Stubenfliege (Musca domestica) oder gegen den Kornkäfer (Sitophilus granarius) oder gegen Schabenarten, wie beispielsweise Blattella germanica oder Periplaneta americana einsetzen.

Darüberhinaus lassen sich die erfindungsgemäßen Wirkstoffe mit besonders gutem Erfolg zur Bekämpfung von parasitisch lebenen Warmblüterschädlingen, wie beispielsweise gegen Räudemilben (Psoroptes ovis) einsetzen.

Daneben weisen die erfindungsgemäßen Wirkstoffe eine starke fungizide Wirkung auf und können zur Bekämpfung von unerwünschten Mikroorganismen praktisch eingesetzt werden. Die Wirkstoffe auch sind für den Gebrauch als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft seien einige Erreger von pilzlichen Krankheiten, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Getreidekrankheiten, wie beispielsweise gegen den Erreger des echten Getreidemehltaues (Erysiphe graminis) oder zur Bekämpfung von Krankheiten im Obst- und Gemüseanbau, wie beispielsweise gegen den Erreger der Tomatenbraunfäule (Phytophthora infestans) oder gegen den Erreger des falschen Rebenmehltaues (Plasmopara viticola) oder zur Bekämpfung von Reiskrankheiten, wie beispielsweise gegen den Erreger der Reisfleckenkrankheit (Pyricularia oryzae) eingesetzt werden.

Darüberhinaus können die erfindungsgemäßen Wirkstoffe in entsprechenden Aufwandmengen auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Mais, Weizen oder Soja einsetzen.

Die Wirkstoffe können in Abhängigkeit von ihren jeweiligen physikalischen und/- oder oder chemischen Eigenschaften in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infragen: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zinn verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetze Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gewichtsprozent Wirkstoff, vorzugsweise zwischen 0,0001 und 1 Gewichtsprozent liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß verwendbaren Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäß verwendbaren Wirkstoffe geschieht auf diesem Gebiet in bekannter Weise, beispielsweise durch orale Anwendung in Form von Tabletten, Kapseln, Tränken oder Granulaten, durch dermale bzw. äußerliche Anwendung in Form beispielsweise des Tauchens (Dippen), Sprühens (Sprayen), Aufgießens (pour-on oder spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke) möglich.

Die erfindungsgemäßen Wirkstoffe können bei der Anwendung als Fungizide ebenfalls in den Formulierungen in Mischung mit anderen bekannten Wirkstoffen vorliegen, wie Fungizide, Insektizide, Akarizide und Herbizide sowie in Mischungen mit Düngemitteln und Wachstumsregulatoren.

Die Wirkstoffe können bei der Anwendung als Fungizide als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, oder Bestreichen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können bei der Anwendung als Fungizide die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden bei der Anwendung als Fungizide im allgemeinen Wirkstoffmengen von 0,001 bis 50 g je Kilogramm Saatgut, vorzugsweise 0,01 bis 10 g benötigt.

Bei der Behandlung des Bodens sind bei der Anwendung als Fungizide Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind. Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfüron, Bensulfuronmethyl, Chlorimuron-ethyl, Chlorsulfuron, Cinosulfuron, Metsulfuron-methyl, Nicosulfüron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfüresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist bei der Anwendung als Herbizide möglich.

Die Wirkstoffe können dabei als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können bei der Anwendung als Herbizide sowohl vor als auch nach dem Auflaufen der Pflanzen appliziert werden. Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

<u>Beispiel II-1</u>:

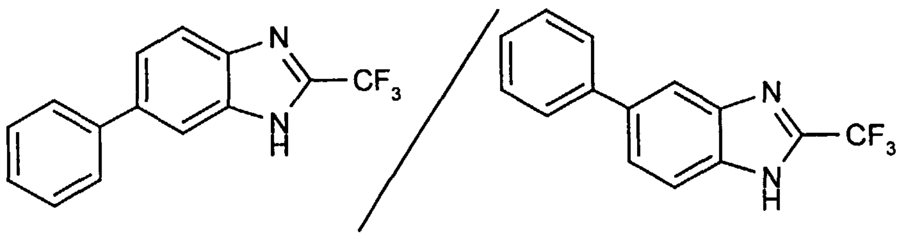

18,4 g (0,092 Mol) 3,4-Diaminobiphenyl werden mit 150 ml Trifluoressigsäure für 5 Stunden auf Rückflußtemperatur erhitzt. Anschließend wird überschüssige Trifluoressigsäure abdestilliert, der Rückstand zwischen 200 ml Essigester und 70 ml Wasser verteilt, die organische Phase abgetrennt, mit jeweils 70 ml gesättigter wässriger Natriumhydrogencarbonatlösung und Wasser gewaschen, über Natriumsulfat getrocknet, im Vakuum eingeengt und der Rückstand durch Chromatographie an Kieselgel (Laufmittel: Cyclohexan/Essigester 2:1) gereinigt.

Man erhält 18,3 g (76 % der Theorie) an 5(6)-Phenyl-2-trifluormethyl-1H-benzimidazol als 1:1 Tautomerengemisch vom Schmelzpunkt 177-182°C.

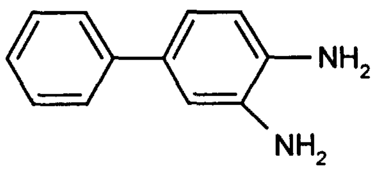

88 g (0,4 Mol) 4-Amino-3-nitro-biphenyl (92-prozentig) werden in 3.000 ml Methanol in Gegenwart von 10 g Raney-Nickel bei 60°C und einem Druck von 5 bar mit molekularem Wasserstoff hydriert. Zur Aufarbeitung wird das Raney-Nickel abfiltriert und das Filtrat im Vakuum eingeengt.

Man erhält 69,2 g (86 % der Theorie) an 3,4-Diaminobiphenyl vom Schmelzpunkt 96-99°C (Reinheit gemäß HPLC 92%).

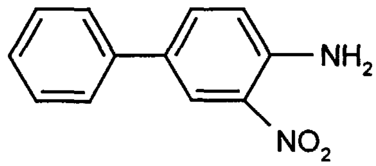

43 g (0,15 Mol) 4-Acetamido-3-nitro-biphenyl (90-prozentig) werden zusammen mit 1,6 g (0,03 Mol) Natriummethylat in 500 ml Methanol für 2 Stunden auf Rückflußtemperatur erhitzt. Zur Aufarbeitung gießt man die abgekühlte Reaktionsmischung in 1.300 ml Eiswasser, rührt 10 Minuten, saugt dann ausgefallenen Niederschlag ab und trocknet ihn.

Man erhält 33 g (94 % der Theorie) an 4-Amino-3-nitro-biphenyl vom Schmelzpunkt 163-165°C (Reinheit gemäß HPLC 92%).

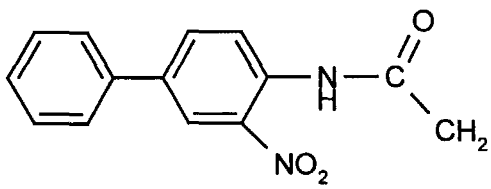

Zu einer Suspension von 84,4 g (0,4 Mol) 4-Acetamido-biphenyl (vergl. z.B. Beilstein Band 12, 4.Ergänzungswerk, S.3248) in 340 ml Eisessig gibt man bei 70°C tropfenweise unter Rühren eine Mischung aus 50,4 ml (1,2 Mol) 98-prozentiger Salpetersäure und 60 ml Eisessig und rührt nach beendeter Zugabe eine weitere Stunde bei 70°C. Zur Aufarbeitung gibt man die abgekühlte Reaktionsmischung in 1.00 ml Eiswasser, rührt 10 Minuten, saugt ausgefallenen Niederschlag ab, wäscht ihn mit 200 ml Wasser und trocknet ihn.

Man erhält 100 g (88 % der Theorie) an 4-Acetamido-3-nitro-biphenyl vom Schmelzpunkt 128-131°C (Reinheit gemäß HPLC 90%).

In entsprechender Weise erhält man die folgenden 1 H-Benzimidazole der Formel

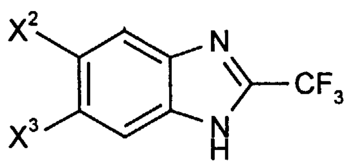

(II)

| Bsp.-Nr. | $X^2$ | $X^3$ | physikalische Eigenschaften |
|---|---|---|---|
| II-a | $CF_3$ (Br) | Br ($CF_3$) | Fp. 209°C |
| II-b | $CF_3O$ (Cl) | Cl ($CF_3O$) | Fp. 144°C |
| II-c | $CF_3O$ (Br) | Br ($CF_3$) | Fp. 161°C |

Als Ausgangsprodukte zur Herstellung der Verbindungen der Formel (I) dienen Fluoralkylgruppen enthaltende o-Phenylendiamine der Formel

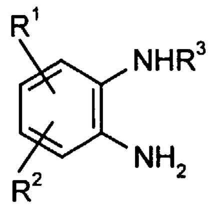

in der

$R^1$ für $CF_3$ oder $OCF_3$ steht,

$R^2$ für Cl oder Br steht,

$R^3$ für Wasserstoff, $COCH_3$ oder $COCF_3$ stehen.

Diese werden erhalten wie in der EP-A 251 013 und der EP-A 487 286 beschrieben oder indem man ein Benzolderivat der Formel

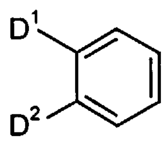

in der

$D^1$ für $CF_3O$ und

$D^2$ für Chlor und Brom
steht

dinitriert, die Nitrogruppen anschließend reduziert und so Verbindungen erhält, bei denen $R^1$ und $R^2$ in 4- und 5-Stellung zu den Aminogruppen stehen und die Bedeutung von $D^1$ und $D^2$ haben.

Sollen Verbindungen hergestellt werden, bei denen $R^1$ die oben angegebene Bedeutung hat und in 4-Stellung zu den Aminogruppen steht und $R^2$ für Cl oder Br in 5-Stellung zu den Aminogruppen steht, so kann man z.B. ein Nitrobenzolderivat der Formel

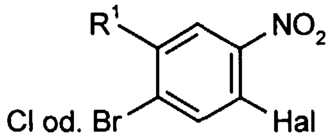

in der

$R^1$ die bei Formel angegebene Bedeutung hat und

Hal für Fluor, Chlor oder Brom steht,

mit Ammoniak umsetzen, so die Hal-Gruppe gegen eine Aminogruppe austauschen und das so erhaltene Nitranilin reduzieren.

Sollen Verbindungen hergestellt werden, bei denen $R^1$ eine Donorgruppe in 4-Stellung zu den beiden Aminogruppen, $R^2$ eine Akzeptorgruppe, z.B. COO-$C_1$-$C_6$-Alkyl, CN, $CF_3$ oder $SO_2$-$C_1$-$C_6$-Alkyl darstellt und $R_3$ ungleich Wasserstoff ist, so kann man z.B. ein Benzolderivat der Formel

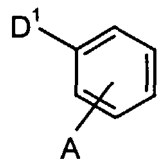

in der

$D^1$ die oben angegebene Bedeutung hat und

A für $CF_3$ steht,

mononitrieren (Eintritt der $NO_2$-Gruppe in para-Position zu $D^1$), die $NO_2$-Gruppe zur $NH_2$-Gruppe reduzieren, die $NH_2$-Gruppe z.B. mit Essigsäure oder Trifluoressigsäure acylieren, nochmals mononitrieren (Eintritt dieser $NO_2$-Gruppe in ortho-Position zur NHCOR-Gruppen mit R = z.B. $CH_3$ oder $CF_3$), diese $NO_2$-Gruppe zur $NH_2$-Gruppe reduzieren und gegebenenfalls, wenn man eine Verbindung der obigen Formel mit $R^3$ = Wasserstoff herstellen will, die Acylgruppe durch Verseifung abspalten.

Die Fluoralkylgruppen enthaltenden o-Phenylendiamine, in denen $R^3$ Wasserstoff bedeutet, können mit Trifluoressigsäure zu 2-Trifluormethylbenzimidazolen der Formel

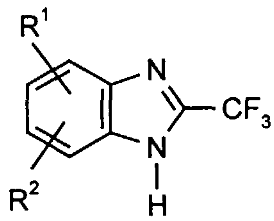

umgesetzt werden.

**<u>Beispiele für Dinitrierung und Reduktion</u>**

<u>Beispiel 1b</u>

Zu 500 g einer Mischsäure enthaltend 33 Gew.-% $HNO_3$ und 67 Gew.-% $H_2SO_4$ wurden 320 g 1,2-Bis-(2-chlor-1,1,2-trifluorethoxy)-benzol getropft. Nach einer Stunde bei 40°C wurden 250 ml 20 gew.-%iges Oleum zugetropft. Anschließend wurde auf 80°C erhitzt und 15 Stunden lang nachgerührt. Dann wurden weitere 120 ml 20 gew.-%iges Oleum und 250 g der oben angegebenen Mischsäure zugetropft. Nach 6 Stunden bei 80 bis 82°C wurde abgekühlt und auf Eis gegossen. Die organische Phase wurde abgetrennt und mit Wasser gewaschen. Nach azeotroper Trocknung mit 1,2-Dichlorethan wurden 350 g 96 Gew.-% reines 1,2-Dinitro-4,5-bis-(2-chlor-1,1,2-trifluorethoxy)-benzol erhalten (Öl, $n_D^{20}$ :1,4832, GC 99,1 %).

350 g dieser Dinitroverbindung wurden zu einem Gemisch aus 1,5 l Ethanol, 50 ml Wasser, 30 ml konzentrierter wäßriger Salzsäure und 470 g Eisenspänen getropft und insgesamt 15 Stunden zum Sieden am Rückfluß erhitzt. Danach wurde die erkaltete Lösung abfiltriert, eingeengt und der Rückstand aus Cyclohexan umkristallisiert. Es wurden 216 g 1,2-Diamino-4,5-bis-(2-chlor-1,1,2-trifluorethoxy)-benzol mit einem Schmelzpunkt von 58 bis 60°C erhalten.

<u>Beispiel 2b</u>

Analog Beispiel 1b wurde aus 1-Trifluormethoxy-2-brombenzol die entsprechende 4,5-Dinitroverbindung

(Schmelzpunkt 73 bis 75°C) und die entsprechende 4,5-Diaminoverbindung (Öl, 98 Gew.-% rein $n_D^{20}$ : 1,5485) hergestellt.

Beispiel 3b

Analog Beispiel 1b wurde aus 1-Trifluormethoxy-2-chlorbenzol die entsprechende 4,5-Dinitroverbindung (Schmelzpunkt 55 bis 56°C) und die entsprechende 4,5-Diaminoverbindung (Schmelzpunkt 56 - 57°C) hergestellt.

**Beispiele für Verdrückung mit Ammoniak und Reduktion**

Beispiel 4b

In einem Autoklaven wurden 260 g 3-Nitro-2,5-dichlorbenzotrifluorid, 130 ml Wasser und 10 g Tetraethylammoniumchlorid vorgelegt und 120 ml flüssiges Ammoniak aufgedrückt. Anschließend wurde auf 130°C erhitzt und für 10 Stunden bei dieser Temperatur gerührt. Nach dem Abkühlen wurde der Ansatz abfiltriert, der abgetrennte Niederschlag mit Wasser gewaschen und getrocknet. Es fielen 194 g 2-Amino-3-nitro-5-chlor-benzotrifluorid mit einem Schmelzpunkt von 67°C an.

134 g des wie oben beschrieben erhaltenen Nitranilins wurden in 800 ml Ethanol gelöst, dann 20 ml Wasser, 10 ml konzentrierte wäßrige Salzsäure und 160 g Eisenspäne zugegeben. Die Mischung wurde für 15 Stunden zum Sieden am Rückfluß erhitzt, dann abgekühlt, abgesaugt, der Filterrückstand mit Dichlormethan gewaschen und anschließend die organischen Phasen unter reduziertem Druck vom Lösungsmittel befreit. Es fielen 171 g 5-Chlor-3-trifluormethyl-1,2-diaminobenzol mit einem Schmelzpunkt von 53°C an.

Beispiel 5b

Analog Beispiel 4b wurde aus 3-Nitro-4,6-dichlor-difluorchlormethoxybenzol zunächst 3-Nitro-4-amino-6-chlor-difluorchlormethoxybenzol (Schmelzpunkt 73 °C) und daraus 3,4-Diamino-6-chlor-difluorchlormethoxybenzol (Öl) erhalten.

**Beispiele für Nitrierung und Reduktion in 2 Stufen**

Beispiel 8

263 g 4-(2,6-Dichlor-4-trifluormethyl)-phenoxy-acetanilid wurden in 1 100 ml Dichlormethan gelöst und bei 10°C vorgelegt. Dann wurden bei dieser Temperatur 88 g 98 Gew.-%ige Salpetersäure zugetropft. Es wurde 1 Stunde bei 10°C und 2 weitere Stunden bei 30°C nachgerührt. Nach der Zugabe von 300 ml Wasser wurden die Phasen getrennt und die organische Phase unter reduziertem Druck vom Dichlormethan befreit. Es verblieben 253 g 2-Nitro-4-(2,6-dichlor-4-trifluormethyl-phenoxy)-acetanilid mit einem Schmelzpunkt von 138 - 140°C.

91 g des so hergestellten Acetanilids wurden in 800 ml Dioxan gelöst, 10 g Raney-Nickel zugegeben und bei 25 bis 45°C in einer Hydrierapparatur mit maximal 50 bar Wasserstoffdruck hydriert. Nach Entspannen und Filtration wurde das Dioxan bei leichtem Vakuum abdestilliert. Es verblieben 65 g 2-Amino-4-(2,6-dichlor-4-trifluormethyl-phenoxy)-acetanilid mit einem Schmelzpunkt von 222 - 223 °C.

Beispiel 9

Analog Beispiel 8 wurde aus 3-Trifluormethyl-4-brom-trifluormethylacetanilid zunächst 3-Trifluormethyl-4-brom-6-nitro-trifluormethylacetanilid (Schmlezpunkt 110 bis 112°C) und daraus 3-Trifluormethyl-4-brom-6-amino-trifluormethylacetanilid (Schmelzpunkt 63 - 65°C) hergestellt.

Beispiel 10

0,2 mol 3-Brom-5-trifluormethyl-phenylen-diamin wurden mit 150 ml Trifluoressigsäure für 3 Stunden auf Rückflußtemperatur erhitzt. Zur Aufarbeitung wurde überschüssige Trifluoressigsäure abdestilliert und der Rückstand zwischen 100 ml Wasser und 300 ml Essigester verteilt. Die organische Phase wurde abgetrennt, nacheinander mit jeweils 100 ml wäßriger Natriumhydrogencarbonatlösung und Wasser gewaschen, über Natriumsulfat getrocknet und im Vakuum eingeengt. Der Rückstand wurde durch Chromatographie an Kieselgel (Laufmittel: Cyclohexan/Essigester 1:1) gereinigt.

Man erhielt 4-Brom-6-trifluormethyl-2-trifluormethyl-1H-benzimidazol vom Schmelzpunkt 149 - 151°C.

## Patentansprüche

1. Substituierte 1 H-Benzimidazole der Formel

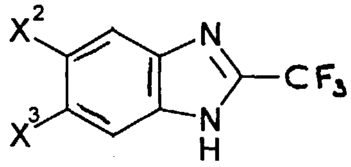

in welcher $X^2$ und $X^3$ folgende Bedeutung haben:

| $X^2$ | $X^3$ |
|---|---|
| $CF_3$ | Br |
| $-OCF_3$ | Cl |
| $-OCF_3$ | Br |

2. Substituiertes Benzimidazol gemäß Anspruch 1 der Formel

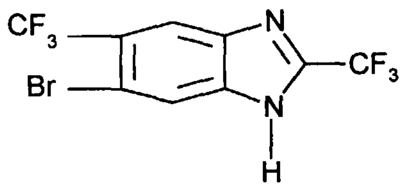

3. Schädlingsbekämpfungsmittel und Mittel zur Bekämpfung tierischer Parasiten auf dem Gebiet der Veterinärmedizin, gekennzeichnet durch einen Gehalt an mindestens einem substituierten Benzimidazol gemäß Anspruch 1 oder 2.

4. Verwendung von substituierten Benzimidazolen gemäß Anspruch 1 oder 2 zur Herstellung von Mitteln zur Bekämpfung von Schädlingen und Parasiten auf dem Gebiet der Veterinärmedizin.

5. Verfahren zur Herstellung von Schädlingsbekämpfungsmitteln, dadurch gekennzeichnet, daß man substituierte Benzimidazole der Formel (I) gemäß Anspruch 1 oder 2 mit Streckmitteln und/oder oberflächenaktiven Mitteln vermischt.

## Claims

1. Substituted 1H-benzimidazoles of the formula

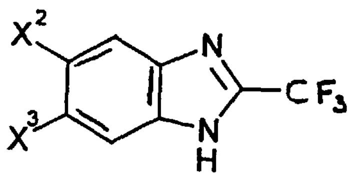

in which $X^2$ to $X^3$ have the following meaning:

| $X^2$ | $X^3$ |
|---|---|
| $CF^3$ | Br |
| $-OCF^3$ | Cl |
| $-OCF^3$ | Br |

**2.** Substituted benzimidazole according to Claim 1, of the formula

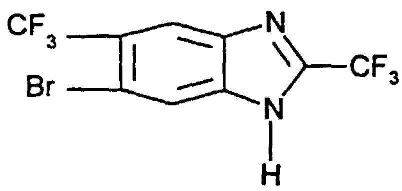

**3.** Pesticides and compositions for combating animal parasites in the field of veterinary medicine, characterized in that they contain at least one substituted benzimidazole according to Claim 1 or 2.

**4.** Use of substituted benzimidazoles according to Claim 1 or 2 for the preparation of compositions for combating pests and parasites in the field of veterinary medicine.

**5.** Process for the preparation of pesticides, characterized in that substituted benzimidazoles of the formula (I) according to Claim 1 or 2 are mixed with extenders and/or surface-active agents.

**Revendications**

**1.** 1H-benzimidazoles substitués de formule

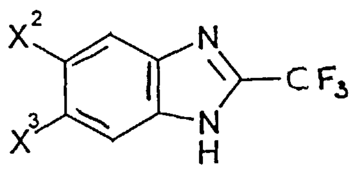

dans laquelle $X^2$ et $X^3$ ont les définitions suivantes :

| $X^2$ | $X^3$ |
|---|---|
| $CF_3$ | Br |
| $-OCF_3$ | Cl |
| $-OCF_3$ | Br |

**2.** Benzimidazole substitué suivant la revendication 1, de formule

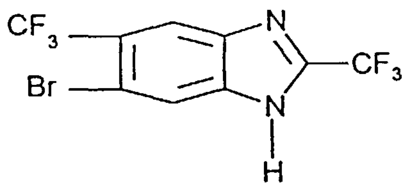

**3.** Compositions pesticides et compositions destinées à combattre des parasites animaux dans le domaine de la médecine vétérinaire, caractérisées par une teneur en au moins un benzimidazole substitué suivant la revendication 1 ou 2.

**4.** Utilisation de benzimidazoles substitués suivant la revendication 1 ou 2 pour la préparation de compositions destinées à combattre des organismes nuisibles et des parasites dans le secteur de la médecine vétérinaire.

**5.** Procédé de préparation de compositions pesticides, caractérisé en ce qu'on mélange des benzimidazoles substitués de formule (I) suivant la revendication 1 ou 2 avec des diluants et/ou des agents tensioactufs.